# EUROPÄISCHE PATENTANMELDUNG

(11) **EP 1 359 155 A1**
(43) Veröffentlichungstag der Anmeldung: **05.11.2003**
(21) Anmeldenummer: 03014348.1
(22) Anmeldetag: 10.08.2000
(51) Int. Cl.: C07K 1/113

(54) **Rückfaltung von Membranproteinen durch Verwendung von Zwei unterschiedlichen Detergentien**

(30) Priorität: 19.08.1999 DE 19939246
(62) Teilanmeldung aus: 00960428.1
(71) Anmelder: M-Phasys GmbH, 72070 Tübingen (DE)
(72) Erfinder: Kiefer, Hans, 70599 Stuttgart (DE); Maier, Klaus, 70599 Stuttgart (DE)
(74) Vertreter: Otten, Hajo, Dr.-Ing.

(57) **Zusammenfassung**

Bei einem Verfahren zur Herstellung von in ihre native Struktur gefalteten Membranproteinen oder Rezeptoren werden zunächst in einem ersten Detergens solubilisierte Proteine bereitgestellt. Um eine Faltung der Proteine in ihre native Form zu induzieren, wird das erste Detergens gegen ein zweites Detergens ausgetauscht. Sowohl für das erste als auch für das zweite Detergens werden Beispiele angegeben.

## Beschreibung

Die vorliegende Erfindung betrifft ein Verfahren zur Herstellung von in ihre native oder aktive Struktur gefalteten Proteinen aus der Gruppe, die Membranproteine, insbesondere Rezeptoren, beispielsweise aus der Familie der G-proteingekoppelten Rezeptoren, sowie Teilsequenzen, homologe Sequenzen, mutierte Sequenzen und abgeleitete Sequenzen der Membranproteine und Rezeptoren umfasst, mit den Schritten:
- Bereitstellen von in einem ersten Detergens solubilisiertem Protein, und
- Austausch des ersten Detergens' gegen ein zweites Detergens, das die Faltung des Proteins in dessen native oder aktive Form induziert.

Für Membranproteine ist ein derartiges Verfahren aus dem Artikel "Refolding of *Escherichia coli* produced membrane protein inclusion bodies immobilised by nickel chelating chromatography", Rogl et al. in FEBS Letters 432 (1998) 21-26, bekannt.

Ein Verfahren zur Rückfaltung von Rezeptorprotein ist aus dem Artikel "Expression of an Olfactory Receptor in *Escherechia coli:* Purification, Reconstitution, and Ligand Binding" von Kiefer et al. in Biochemistry 35 (1996) 16077-16084, bekannt.

Den beiden Veröffentlichungen liegt das Problem zugrunde, dass Membranproteine, zu denen auch die Rezeptoren gehören, zwar in großer Menge mit Hilfe von Expressionsvektoren in Bakterien produziert werden können, dass das produzierte Protein jedoch nicht "aktiv" ist. Das Protein wird nämlich nicht in die Membran integriert sondern liegt zunächst im denaturierten Zustand vor und muss durch einen Detergensaustausch in die native oder aktive Struktur (rück)gefaltet werden. Die Aggregate von "inaktivem" Protein werden in der englischsprachigen Literatur als inclusion bodies bezeichnet.

Für die Membranproteine Toc75 und LHCP beschreiben Rogl et al. ein Verfahren, bei dem als erstes Detergens N-Lauroylsarcosin und als zweites Detergens Triton X-100® verwendet wurden. Durch den Austausch des chaotropen durch das milde Detergens ergab sich eine Rückfaltung des aggregierten Proteins.

Gemäß Kiefer et al. wurde ein G-proteingekoppelter Geruchsrezeptor durch Detergensaustausch von N-Lauroylsarcosin in Digitonin während der Bindung an eine Nickelsäule in die aktive Struktur überführt.

In beiden Fällen konnte gezeigt werden, dass das zunächst in Form von inclusion bodies vorliegende aggregierte Protein zunächst in einem denaturierenden Detergens solubilisiert und dann durch den beschriebenen Detergensaustausch in seine aktive Struktur überführt werden konnte, die durch entsprechende Bindungsmessungen verifiziert wurde.

An Membranproteinen, insbesondere an Rezeptoren in nativer oder aktiver Form besteht nicht nur wissenschaftliches sondern auch großes kommerzielles Interesse, denn die Membranproteine sind Bestandteile aller biologischen Membranen und verleihen den verschiedenen zellulären Membranen ihre Spezifität, sind insbesondere für den Stoff- und Reizaustausch verantwortlich.

Die spezifische Erkennung einer chemischen Verbindung durch den zugehörigen Rezeptor hat z.B. zur Folge, dass die Zielzelle ihren physiologischen Zustand ändert. Darum sind Rezeptoren die wichtigsten Zielmoleküle für Medikamente, ca. 3/4 aller im Handel befindlichen Pharmaka wirken auf Rezeptoren, die meisten davon wiederum auf sogenannte G-proteingekoppelte Rezeptoren, die im menschlichen Genom mehrere hundert Vertreter haben.

Für die Entwicklung von spezifischen Antikörpern, von Medikamenten etc. ist es vor diesem Hintergrund sehr wünschenswert, Membranproteine, insbesondere Rezeptoren in aktiver oder nativer Struktur in großen Mengen zur Verfügung zu haben. Da diese Proteine im Gewebe jedoch nur in sehr geringer Konzentration vorkommen, ist es erforderlich, ein System zur rekombinanten Überexpression der Membranproteine und Rezeptoren einzusetzen. Hierzu kann zum einen in eukaryotischen Zellen (Säuger- oder Insektenzellen) funktionelles Protein erzeugt werden, die System sind jedoch teuer, und die Expressionsraten sind niedrig, was ebenfalls von Nachteil ist. Auch bei der bakteriellen Expression kann man funktionelles Protein erhalten, allerdings ist hier die Expressionsrate in der Regel noch niedriger als bei eukaryotischer Expression.

Vor diesem Hintergrund beschreiben die beiden eingangs erwähnten Veröffentlichungen Verfahren, bei denen das Protein im Zellinneren exprimiert wird, wo es jedoch aggregiert, also nicht funktionell vorliegt. Der Vorteil dieser Methode liegt darin, dass sehr große Mengen an Protein hergestellt werden können, Kiefer et al. berichten, dass bis zu 10 % des Zellproteins und damit 100-10.000 mal mehr Protein als mit anderen Expressionssystemen hergestellt werden kann. Die dabei erzeugten inclusion bodies, über die auch Rogl et al. berichten, müssen dann zunächst solubilisiert und durch den eingangs bereits beschriebenen Detergensaustausch in ihre native oder aktive Struktur überführt werden.

Selbstverständlich richtet sich das kommerzielle Interesse nicht nur auf Membranproteine und Rezeptoren in ihrer natürlich vorkommenden Sequenz, vielmehr sind auch Teilsequenzen, homologe Sequenzen, mutierte Sequenzen oder abgeleitete Sequenzen von Membranproteinen und Rezeptoren Gegenstand dieser Erfindung, denn sie ermöglichen je nach Funktionalität nicht nur Einblicke in die Struktur von Membranproteinen und Rezeptoren, sondern auch ein rationales Medikamentendesign.

In diesem Zusammenhang sei erwähnt, dass die DNA-Sequenz für viele Rezeptoren bekannt ist, derartige Sequenzen sind in der EMBL-Datenbank enthalten. Da diese DNA-Sequenzen meistens keine Introns enthalten, lässt sich die kodierende Sequenz über PCR aus genomischer DNA oder über RT-PCR aus mRNA herstellen. Diese DNA kann dann in einen entsprechenden Expressionsvektor kloniert werden.

Unbekannt ist jedoch die Struktur des Translationsproduktes, so dass die Bereitstellung von erfindungsgegenständlichen Proteinen in ausreichender Menge Kristallisationsexperimente etc. ermöglicht, um die Struktur weiter aufzuklären.

Es sei noch erwähnt, dass eukaryotisch und bakteriell exprimierte Rezeptoren durch die Glykosylierung unterschieden werden können. G-proteingekoppelte Rezeptoren besitzen nämlich am N-Terminus eine oder mehrere Glykosylisierungsstellen, die im endoplasmatischen Retikulum oder später in Golgi-Apparat mit einem Oligosaccharid modifiziert werden. Bakterien modifizieren diese Sequenzen dagegen nicht.

Durch Behandlung eines Teil des Proteins mit N-Glykosidase F oder N-Glykosidase A kann der Saccharidanteil abgespalten werden, so dass auf einem SDS-Gel eine unterschiedliche Lauflänge für Protein vor und nach dieser Behandlung zu erkennen ist, wenn das Protein in eukaryotischen Zellen exprimiert wurde. Bei bakteriell exprimiertem Protein sind keine Lauflängenunterschiede erkennbar.

Obwohl die in den eingangs erwähnten Veröffentlichungen beschriebenen Verfahren zur Herstellung von Membranprotein bzw. Rezeptorprotein zu aktiven Strukturen führen, sind die beschriebenen Verfahren nach Erkenntnis der Erfinder der hier vorliegenden Anmeldung insofern nicht zufriedenstellend, als die Ausbeute niedrig und das Verfahren schlecht reproduzierbar ist.

Vor diesem Hintergrund ist es Aufgabe der vorliegenden Erfindung, das eingangs erwähnte Verfahren dahingehend weiterzubilden, dass bei guter Reproduzierbarkeit eine hohe Ausbeute des Proteins in aktiver oder nativer Struktur erreicht wird.

Erfindungsgemäß wird diese Aufgabe bezüglich der eingangs genannten Herstellung von Proteinen, außer der von G-proteingekoppelten Rezeptoren, dadurch gelöst, dass das zweite Detergens ausgewählt ist aus der Gruppe:
- Alkyl-N,N-dimethylglycin (Alkyl = C8-C16)
- Alkylglykoside (Alkyl = C5-C12, auch verzweigtkettige oder cyclische Alkylreste, glycosid = Alle Mono- und Disaccharide)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin)
- Alkyl-Phosphorylcholin (Alkyl = C10 - C16).

Bezüglich der eingangs genannten Herstellung von G-proteingekoppelten Rezeptoren wird diese Aufgabe dadurch gelöst, dass das zweite Reagenz ausgewählt ist aus der Gruppe:
- Alkyl-N,N-dimethylglycin (Alkyl = C8-C16)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin).

Die der Erfindung zugrundeliegende Aufgabe wird auf diese Weise vollkommen gelöst.

Die Erfinder der vorliegenden Anmeldung haben nämlich erkannt, dass die geringe Ausbeute und mangelnde Reproduzierbarkeit bei den bekannten Verfahren auf das zweite Detergens zurückzuführen ist. Überraschenderweise ergeben sich nämlich deutlich höhere Ausbeuten und reproduzierbarere Ergebnisse, wenn das zweite Detergens aus der oben erwähnten Gruppe ausgewählt ist. Dabei ist darauf zu achten, dass das zweite Detergens in seiner Endkonzentration oberhalb der kritischen micellaren Konzentration liegt. Dieser cmc-Wert gibt die Konzentration einer amphiphilen Micellen-bildenden Struktur in Wasser an, oberhalb derer sich Micellen bilden. Der cmc-Wert spiegelt also im Prinzip die Löslichkeit eines Detergens' in Wasser wieder. Oberhalb des cmc-Wertes ist die Konzentration gelöster Detergens-Monomere konstant.

Die cmc-Werte einiger Detergentien sind beschrieben in der Veröffentlichung "Detergents: An Overview" J.M. Neugebauer in Methods in Enzymology 182 (1990), Seiten 239-253.

Dabei ist es besonders bevorzugt, wenn bei der Herstellung der eingangs genannten Proteine, außer bei den G-proteingekoppelten Rezeptoren, als zweites Detergens Alkyl-Phosphorylcholin mit einer Kettenlänge von C10-C16 eingesetzt wird. Die Erfinder der vorliegenden Anmeldung haben erkannt, dass insbesondere bei solchen Rezeptoren dieses Detergens für eine hohe Proteinausbeute in rückgefalteter Struktur sorgt.

Gemäß eigener Messungen der Erfinder betragen die cmc-Werte für Alkyl-Phosphorylcholin mit einem Alkylrest von C12, C13, C14 bzw. C16 500, 150, 50 bzw. 5 µM.

Angesichts der Tatsache, dass nur wenige Detergentien überhaupt in der Lage sind, Membranproteine oder gar Rezeptoren stabil in Lösung zu halten, ist es umso überraschender, dass Alkyl-Phosphorylcholin, dessen Verwendung für Rezeptoren bisher in der Literatur nicht beschrieben wurde, sogar in der Lage ist, eine Rückfaltung in die native Struktur zu induzieren. Im Falle eines Adenosinrezeptors konnten die Erfinder nachweisen, dass der rückgefaltete Rezeptor native Bindungseigenschaften aufweist, wenn als zweites Detergens Alkyl-Phosphorylcholin eingesetzt wird. Auch für andere Rezeptoren konnte die Rückfaltung mit einem der Detergentien aus der oben genannten Gruppe gezeigt werden.

Dabei ist es bevorzugt, wenn die Proteine, einschließlich der G-proteingekoppelten Rezeptoren, in Form von inclusion bodies in einer mit einem Expressionsvektor transformierten Zellinie produziert wird, in den ein für das Protein kodierendes Gen kloniert ist, wobei die Proteine vorzugsweise Teile von Fusionsproteinen sind und vor oder nach dem Detergensaustausch von den Fusionsproteinen abgespalten werden.

Die Expression der für die erfindungsgegenständlichen Proteine kodierenden DNA-Sequenzen als Fusionsproteine hat gegenüber der direkten Expression ohne Trägerprotein den Vorteil, dass dieses das gewünschte, jedoch expressionssystemfremde Protein vor Abbau durch Proteasen schützt und zu einem höheren Expressionsniveau führen kann. Insbesondere durch die Verwendung von Glutathion-S-Transferase (GST) als Trägerprotein wird die Löslichkeit von in großen Mengen exprimierten Proteinen in der Wirtszelle erhöht und die Isolierung erleichtert. Das Trägerprotein kann ferner zur Reinigung von Fusionsproteinen benutzt werden, wenn geeignete Antikörper vorliegen. Gleiches gilt für affinitätschromatographische Reinigungsverfahren.

Dabei ist es weiter bevorzugt, wenn die inclusion bodies aufgereinigt und durch Zusatz des ersten Detergens solubilisiert werden, wobei das erste Detergens ausgewählt ist aus der Gruppe:

N-Lauroylsarcosin, Dodecylsulfat, andere geladene Detergentien oder Harnstoff bzw. Guanidiniumchlorid in Kombination mit geladenen oder ungeladenen Detergentien.

Wichtig ist dabei, dass die Bedingungen, um das Protein in Lösung zu bringen, denaturierend sind, so dass sie die Ausbildung der nativen Struktur nicht ermöglichen.

Dabei ist es insgesamt bevorzugt, wenn das zweite Detergens in einem Faltungspuffer mit gemischten Lipid/Detergensmicellen vorliegt, wobei der Faltungspuffer vorzugsweise das zweite Detergens und Phospholipid aus einer natürlichen Quelle, vorzugsweise einen Lipidextrakt aus Gewebe enthält, in dem das Protein natürlicherweise vorkommt.

Hierbei ist von Vorteil, dass gegenüber der Verwendung von reinen Detergensmicellen die Ausbeute an nativem Protein noch verbessert werden kann. Den Lipidextrakt aus dem Gewebe, in dem der Rezeptor natürlicherweise vorkommt, kann man auch simulieren, indem Lipide mit einer ähnlichen Zusammensetzung verwendet oder gemischt werden.

Bezüglich des Detergensaustausches ist es bevorzugt, wenn dieser durch Dialyse- oder Ultrafiltrationsverfahren, bzw. über chromatographische Verfahren oder durch Verdünnen des solubilisierten Proteins in einen das zweite Detergens enthaltenden Puffer erfolgt.

Die insoweit beschriebenen Verfahren zum Detergensaustausch sind untereinander austauschbar und bieten jedes für sich spezifische Vorteile bezüglich der Handhabung, der Verfahrensdauer sowie der erreichbaren Ausbeute.

Nach dem Detergensaustausch muss in dem Protein noch zumindest eine konservierte Disulfidbrücke ausgebildet werden, was vorzugsweise durch Zugabe einer Mischung aus oxidiertem und reduziertem Glutathion erfolgt.

Das gefaltete Protein kann ferner in Proteoliposomen eingebaut werden, die künstlich hergestellte Vesikel sind und eine funktionstüchtige Einheit darstellen. Mit Hilfe dieser gezielt hergestellten Proteoliposomen können bestimmte Prozesse an den Membranproteinen/Rezeptoren gezielt untersucht werden.

Vor diesem Hintergrund betrifft die vorliegende Erfindung ferner Proteoliposomen mit nach dem obigen Verfahren hergestelltem Protein.

Ferner betrifft die vorliegende Erfindung die Verwendung eines Detergens zur Herstellung von in ihre native oder aktive Struktur gefalteten Proteinen der oben beschriebenen Art, ausgenommen von G-proteingekoppelten Rezeptoren, wobei das zweite Detergens ausgewählt ist aus der Gruppe:
- Alkyl-N,N-dimethylglycin (Alkyl = C8-C16)
- Alkylglykoside (Alkyl = C5-C12, auch verzweigtkettige oder cyclische Alkylreste, glycosid = Alle Mono- und Disaccharide)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin)
- Alkyl-Phosphorylcholin (Alkyl = C10 - C16).

Gegenstand der vorliegenden Erfindung ist darüber hinaus auch die Verwendung eines Detergens zur Herstellung von in ihre native oder aktive Struktur gefalteten Proteine aus der Familie der G-proteingekoppelten Rezeptoren, wobei das Detergens ausgewählt ist aus der Gruppe:
- Alkyl-N,N-dimethylglycin (Alkyl = C8-C16)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin).

Weitere Vorteile ergeben sich aus der nachstehenden Beschreibung bevorzugter Ausführungsbeispiele.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand der Beschreibung einzelner Beispiele näher erläutert.

### Beispiel 1: Herstellen eines Expressionsvektors mit cDNA für Rezeptorprotein

Die DNA-Sequenzen für diverse Rezeptorproteine und auch Membranproteine sind in der EMBL-Datenbank enthalten, sie weisen zumeist keine Introns auf. Mit Hilfe von Primern kann also die erforderliche DNA über PCR aus genomischer DNA oder über RT-PCR aus mRNA hergestellt werden.

Diese DNA wird dann in einen Expressionvektor kloniert, der zur Expression eines Fusionsproteines konstruiert wurde. Das Trägerprotein kann bspw. Glutathion-S-Transferase (GST) sein, wie dies in dem eingangs erwähnten Artikel von Kiefer et al. beschrieben ist, wo ein Fusionsprotein aus dem Rezeptor OR5 und GST erzeugt wurde. Der Expressionsvektor wird in eine Zellinie transformiert, die das Fusionsprotein exprimiert. Das Protein wird dabei nicht in die Membran eingebaut, sondern liegt zumindest zum Teil aggregiert in Form von inclusion bodies in Zytplasma vor und ist somit nicht korrekt gefaltet.

### Beispiel 2: Isolierung von exprimiertem Protein

Die cDNA eines der folgenden Rezeptoren wird in-frame in den Vektor pGEX2a-c-His kloniert: AO-Adenosinrezeptors aus dem Hai Squalus acanthias, menschlicher beta-2-adrenerger Rezeptor, menschlicher Neuropeptid YY1-Rezeptor, menschlicher Neuropeptid YY2-Rezeptor, menschlicher Melanocortin-1-Rezeptor, menschlicher Oxytocinrezeptor. Dieser Vektor enthält hinter dem Tac-Promotor die Sequenz, die für Glutathion-S-Transferase und eine darauffolgende Thrombinspaltstelle kodiert, dann eine Polylinkersequenz und schließlich sechs Histidincodons und ein Stopcodon.

Die Vektoren werden in den E. coli-Stamm BL21 transformiert. Die Proteinexpression wird durch Zugabe von IPTG induziert und die Zellen werden nach weiteren drei Stunden geerntet. Nach Lysozymbehandlung und Ultraschallaufschluss werden die Membranen und inclusion bodies durch Zentrifugation von den löslichen Proteinen abgetrennt.

### Beispiel 3: Solubilisierung des Proteins und säulenchromatographischer Detergensaustausch

Die inclusion bodies werden durch Zugabe von 1,5 % N-Lauroylsarcosin bei 0°C solubilisiert und mit einem Puffer (0,1 % Alkyl(C14)phosphorylcholin) auf das fünffache Volumen verdünnt. Zu dieser Lösung gibt man Thrombin und inkubiert 16 Stunden bei 20°C, um den Rezeptor von GST abzuspalten. Anschließend werden unlösliche Zellbestandteile abzentrifugiert.

Der Überstand wird auf Ni-NTA-Agarose (Qiagen) gegeben und eine Stunde lang bei 4°C inkubiert, wobei der Rezeptor an die Nickelmatrix bindet. Danach überführt man das Nickelmaterial in eine Säule und wäscht zum Detergensaustausch mit einem Puffer, der 0,01 % Alkyl(C14)phosphorylcholin als zweites Detergens enthält. Dadurch werden das N-Laurolylsarcosin (erstes Detergens) und kontaminierende Proteine entfernt.

### Beispiel 4: Rekonstitution des Proteins

Zur Rekonstitution löst man eine Lipidmischung, die aus 70 % 1-Palmitoyl-2-oleoylphosphatidylcholin und 30% 1-Palmitoyl-2-oleoylphosphatidylglycerol besteht, zusammen mit der doppelten (w:w) Menge Dodecylmaltosid in Chloroform und entfernt das Lösungsmittel im Vakuum. Darauf gibt man das gereinigte Protein aus Beispiel 3 und inkubiert mindestens eine Stunde lang. Das Detergens wird über eine Polystyrensäule (Calbiosorb von Calbiochem) entfernt, worauf sich Liposomen mit inkorporiertem Rezeptor bilden (Proteoliposomen).

Durch Ligandenbindungsmessungen konnte gezeigt werden, dass der Rezeptor in nativer Struktur vorliegt.

### Beispiel 5: Detergensaustausch mit Lipid/Detergensmicellen

Es werden folgende Stammlösungen angesetzt:
- Cholesterol, Sigma C8667, 100 mg/ml in CHCl₃
- Schafhirnphospholipid, Sigma P4264, 100 mg/ml in CHCl₃
- Sojabohnenlecithin, Sigma P3644, 100 mg/ml in CHCl₃
- 100 mg von Alkyl(C16)-Phosphorylcholin in 50 ml-Fläschen, gelöst in 1-2 ml CHCl₃; Zugabe von 28 µg Cholesterolstammlösung, 32 µl Schafhirnphospholipid-Stammlösung, 40 µl Sojabohnenlecithin-Stammlösung; CHCl₃ abdampfen und wenigstens 30 Minuten bei weniger als 15 mbar trocknen; Zugabe von 1 ml Wasser, um eine klare Lösung zu erhalten (Detergens-Stammlösung, 100 mg/ml)
- Thrombin, Sigma T4648, 1.000 u/ml in H₂O, gelagert bei - 20°C
- Sarcosyl: 10 % N-Lauroylsarcosin in H₂O, autoklaviert
- 10 x PBS: 200 mM Natriumphosphat, 1,5 M NaCl, pH 7,0

2 ml 3 % Sarcosyl werden in PBS aufgenommen und auf Eis gelagert. 2 ml inclusion bodies aus Beispiel 2 zugeben und schütteln sowie eine Minute mit Ultraschall behandeln. Unmittelbar danach 16 ml 0,1 % Detergens-Stammlösung in PBS hinzugeben.

Bereits hier erfolgt ein Detergensaustausch, Sarcosyl wird unter den cmc-Wert verdünnt, während die Endkonzentration von Alkyl(C16)phosphorylcholin oberhalb des cmc-Wertes liegt.

Nach Zugabe von 15 u Thrombin wird die Lösung über Nacht bei 20°C gehalten, um das Fusionsprotein zu spalten.

Danach wird die Lösung für 30 Minuten bei 4°C mit 40.000 UPM zentrifugiert und der Überstand abgenommen.

Zu dem Überstand werden 20 mM Imidazol aus einer 1 M-Stammlösung (pH 7,0) hinzugegeben. Diese Lösung wird zu entsprechend aufgereinigtem Säulenmaterial Ni-NTA superflow (Qiagen) hinzugegeben und in einem Kühlraum (4-8°C) für eine Stunde mäßig rotiert, um ein Absetzen des Materials zu verhindern. Auf diese Weise bindet das Rezeptorprotein an das Säulenmaterial.

Danach wird das Säulenmaterial für eine Minute bei 2.000 UPM zentrifugiert und der Überstand soweit entfernt, dass der verbleibende Überstand dem Bettvolumen entspricht. Ni-NTA Agarose wird aufgenommen und in eine Säule gegeben. Mit einer Flussrate von 2 ml/min wird mit 40 ml einer 0,01 % Detergens-Stammlösung in PBS gewaschen, wodurch ein weiterer Detergensaustausch unter Beachtung der cmc-Werte erfolgt.

Die Säule wird dann mit 10 ml von PBS/0,01 % Detergens-Stammlösung/0,3 M Imidazol eluiert und die bei 280 nm absorbierenden Fraktionen gesammelt.

Daraufhin erfolgt für vier Stunden eine Dialyse gegen 2 1 PBS bei 4°C, sowie Zugabe von 1 mM GSH/0,1 mM GSSG aus einer 100 x Stammlösung in Wasser.

Die Lösung wird dann für 48 Stunden bei 4°C gelagert, woraufhin durch Flussdialyse die Adenosinbindung nachweisbar war, der Rezeptor lag also in nativer Form vor.

### Beispiel 6: Rückfaltung des beta-2-adrenergen Rezeptors durch Detergensaustausch ohne Reinigung des Proteins

Die inclusion bodies mit enthaltenem beta-2-adrenergen Rezeptor aus Beispiel 2 werden durch Zugabe von 1,5% N-Lauroylsarcosin bei 0°C solubilisiert und mit dem 10fachen Volumen einer Lösung von 0,1% Dodecyl-β-D-maltosid in 20mM Na-Malonatpuffer pH 6,0 verdünnt. Anschliessend wird Thrombin (50 Einheiten pro Milligramm Protein) zugegeben und eine Stunde lang bei 20°C inkubiert. Nach Zentrifugation gibt man den Überstand auf einen Lipidfilm wie in Beispiel 4 und rekonstituiert das Protein in Proteoliposomen.

Die erfolgreiche Rückfaltung wird durch Messung der Bindung eines fluoreszierenden Liganden des beta-adrenergen Rezeptors (BODIPY-TMR-CGP 12177 von Molecular Probes) nachgewiesen.

## Patentansprüche

1. Verfahren zur Herstellung von in ihre native oder aktive Struktur gefalteten Proteinen aus der Gruppe, die Membranproteine, insbesondere Rezeptoren, ausgenommen solche aus der Familie der G-proteingekoppelten Rezeptoren, sowie Teilsequenzen, homologe Sequenzen, mutierte Sequenzen und abgeleitete Sequenzen der Membranproteine und Rezeptoren umfasst, mit den Schritten:
- Bereitstellen von in einem ersten Detergens solubilisiertem Protein, und
- Austausch des ersten Detergens' gegen ein zweites Detergens, das eine Faltung des Proteins in dessen native oder aktive Form induziert,
**dadurch gekennzeichnet, dass** das zweite Detergens ausgewählt ist aus der Gruppe:
- Alkyl-N.N-dimethylglycin (Alkyl = C8-C16)
- Alkylglykoside (Alkyl = C5-C12, auch verzweigtkettige oder cyclische Alkylreste, glycosid = Alle Mono- und Disaccharide)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin)
- Alkyl-Phosphorylcholin (Alkyl = C10 - C16).

2. Verfahren nach Anspruch 1, **dadurch gekennzeichnet, dass** das zweite Detergens in einem Faltungspuffer mit gemischten Lipid/Detergensmicellen vorliegt.

3. Verfahren nach Anspruch 2, **dadurch gekennzeichnet, dass** der Faltungspuffer das zweite Detergens und Phospholipid aus einer natürlichen Quelle, vorzugsweise einen Lipidextrakt aus Gewebe enthält, in dem das Protein natürlicherweise vorkommt.

4. Verfahren nach Anspruch 1, **dadurch gekennzeichnet, dass** der Detergensaustausch durch Dialyse- oder Ultrafiltrationsverfahren erfolgt.

5. Verfahren nach Anspruch 1, **dadurch gekennzeichnet, dass** der Detergensaustausch über ein chromatographisches Verfahren erfolgt.

6. Verfahren nach Anspruch 1, **dadurch gekennzeichnet, dass** der Detergensaustausch durch Verdünnen des solubilisierten Proteins in einen das zweite Detergens enthaltenden Puffer erfolgt.

7. Verfahren nach einem der Ansprüche 1 bis 6, **dadurch gekennzeichnet, dass** nach dem Detergensaustausch in dem Protein zumindest eine konservierte Disulfidbrücke ausgebildet wird, vorzugsweise durch Zugabe einer Mischung aus oxidiertem und reduziertem Glutathion.

8. Verfahren nach einem der Ansprüche 1 bis 7, **dadurch gekennzeichnet, dass** das gefaltete Protein in Proteoliposomen eingebaut wird.

9. Verfahren nach einem der Ansprüche 1 bis 8, **dadurch gekennzeichnet, dass** das Protein in Form von inclusion bodies in einer mit einem Expressionsvektor transformierten Zellinie produziert wird, in den ein für das Protein kodierendes Gen kloniert ist.

10. Verfahren nach einem der Ansprüche 1 bis 9, **dadurch gekennzeichnet, dass** das Protein Teil eines Fusionsproteins ist und vor oder nach dem Detergensaustausch von dem Fusionsprotein abgespalten wird.

11. Verfahren nach Anspruch 9, **dadurch gekennzeichnet, dass** die inclusion bodies aufgereinigt und durch Zusatz des ersten Detergens solubilisiert werden.

12. Verfahren nach einem der Ansprüche 1 bis 11, **dadurch gekennzeichnet, dass** das erste Detergens ausgewählt ist aus der Gruppe N-Lauroylsarcosin, Dodecylsulfat, andere geladene Detergentien oder Harnstoff bzw. Guanidiniumchlorid in Kombination mit geladenen oder ungeladenen Detergentien.

13. Verfahren nach einem der Ansprüche 1 bis 12, **dadurch gekennzeichnet, dass** das zweite Detergens oberhalb der kritischen micellaren Konzentration eingesetzt wird.

14. Verfahren nach einem der Ansprüche 1 bis 13, **dadurch gekennzeichnet, dass** als zweites Detergens Alkyl-Phosphorylcholin mit einer Kettenlänge von C10-C16 eingesetzt wird.

15. Proteoliposomen mit nach dem Verfahren nach einem der Ansprüche 1 bis 14 hergestelltem Protein.

16. Verfahren zur Herstellung von in ihre native oder aktive Struktur gefalteten Proteinen aus der Familie der G-proteingekoppelten Rezeptoren mit den Schritten:
- Bereitstellen von in einem ersten Detergens solubilisiertem Protein, und
- Austausch des ersten Detergens' gegen ein zweites Detergens, das eine Faltung des Proteins in dessen native oder aktive Form induziert,
**dadurch gekennzeichnet, dass** das zweite Detergens ausgewählt ist aus der Gruppe:
- Alkyl-N,N-dimethylglycin (Alkyl = C8-C16)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin).

17. Verfahren nach Anspruch 16, **dadurch gekennzeichnet, dass** das zweite Detergens in einem Faltungspuffer mit gemischten Lipid/Detergensmicellen vorliegt.

18. Verfahren nach Anspruch 17, **dadurch gekennzeichnet, dass** der Faltungspuffer das zweite Detergens und Phospholipid aus einer natürlichen Quelle, vorzugsweise einen Lipidextrakt aus Gewebe enthält, in dem das Protein natürlicherweise vorkommt.

19. Verfahren nach Anspruch 16, **dadurch gekennzeichnet, dass** der Detergensaustausch durch Dialyse- oder Ultrafiltrationsverfahren erfolgt.

20. Verfahren nach Anspruch 16, **dadurch gekennzeichnet, dass** der Detergensaustausch über ein chromatographisches Verfahren erfolgt.

21. Verfahren nach Anspruch 16, **dadurch gekennzeichnet, dass** der Detergensaustausch durch Verdünnen des solubilisierten Proteins in einen das zweite Detergens enthaltenden Puffer erfolgt.

22. Verfahren nach einem der Ansprüche 16 bis 21, **dadurch gekennzeichnet, dass** nach dem Detergensaustausch in dem Protein zumindest eine konservierte Disulfidbrücke ausgebildet wird, vorzugsweise durch Zugabe einer Mischung aus oxidiertem und reduziertem Glutathion.

23. Verfahren nach einem der Ansprüche 16 bis 22, **dadurch gekennzeichnet, dass** das gefaltete Protein in Proteoliposomen eingebaut wird.

24. Verfahren nach einem der Ansprüche 16 bis 23, **dadurch gekennzeichnet, dass** das Protein in Form von inclusion bodies in einer mit einem Expressionsvektor transformierten Zellinie produziert wird, in den ein für das Protein kodierendes Gen kloniert ist.

25. Verfahren nach einem der Ansprüche 16 bis 24, **dadurch gekennzeichnet, dass** das Protein Teil eines Fusionsproteins ist und vor oder nach dem Detergensaustausch von dem Fusionsprotein abgespalten wird.

26. Verfahren nach Anspruch 24, **dadurch gekennzeichnet, dass** die inclusion bodies aufgereinigt und durch Zusatz des ersten Detergens solubilisiert werden.

27. Verfahren nach einem der Ansprüche 16 bis 26, **dadurch gekennzeichnet, dass** das erste Detergens ausgewählt ist aus der Gruppe N-Lauroylsarcosin, Dodecylsulfat, andere geladene Detergentien oder Harnstoff bzw. Guanidiniumchlorid in Kombination mit geladenen oder ungeladenen Detergentien.

28. Verfahren nach einem der Ansprüche 16 bis 27, **dadurch gekennzeichnet, dass** das zweite Detergens oberhalb der kritischen micellaren Konzentration eingesetzt wird.

29. Proteoliposomen mit nach dem Verfahren nach einem der Ansprüche 16 bis 28 hergestelltem Protein.

30. Verwendung eines Detergens zur Herstellung von in ihre native oder aktive Struktur gefalteten Proteinen aus der Gruppe, die Membranproteine, insbesondere Rezeptoren, ausgenommen solche aus der Familie der G-prod ngekoppelten Rezeptoren, sowie Teilsequenzen, homologe Sequenzen, mutierte Sequenzen und abgeleitete Sequenzen der Membranproteine und Rezeptoren umfasst, **dadurch gekennzeichnet, dass** das Detergen ausgewählt ist aus der Gruppe:
- Alkyl-N,N-dimethylglycin (Alkyl = C8-C16)
- Alkylglykoside (Alkyl = C5-C12, auch verzweigtkettige oder cyclische Alkylreste, glycosid = Alle Mono- und Disaccharide)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin)
- Alkyl-Phosphorylcholin (Alkyl = C10 - C16).

31. Verwendung eines Detergens' zur Herstellung von in ihre native oder aktive Struktur gefalteten Proteinen aus der Familie der G-proteingekoppelten Rezeptoren, **dadurch gekennzeichnet, dass** das Detergens ausgewählt ist aus der Gruppe:
- Alkyl-N,N-dimethylglycin (Alkyl = C8-C16)
- Saccharid-Fettsäureester (bspw. Sucrosemonododecanoat)
- Alkylthioglycoside (Alkyl = C5-C12, auch verzweigtkettige oder cyclische Alkylreste, glycosid = Alle Mono- und Disaccharide mit S- statt O-glycosidischer Bindung)
- Gallensäuren (Cholat, Deoxycholat) und Derivate (bspw. CHAPS, CHAPSO)
- Glucamide (MEGA-8 bis -10, HEGA)
- Lecithine und Lysolecithine (bspw. DHPC, C12-Lysolecithin).
